# EUROPÄISCHE PATENTSCHRIFT

(11) **EP 3 355 951 B1**
(45) Veröffentlichungstag und Bekanntmachung des Hinweises auf die Patenterteilung: **21.04.2021**
(21) Anmeldenummer: 16774453.1
(22) Anmeldetag: 28.09.2016
(51) Int. Cl.: A61M 1/16

(54) **DIALYSEGERÄT**
DIALYSIS MACHINE
APPAREIL DE DIALYSE

(30) Priorität: 28.09.2015 DE 102015012604
(43) Veröffentlichungstag der Anmeldung: 08.08.2018
(73) Patentinhaber: Fresenius Medical Care Deutschland GmbH, 61352 Bad Homburg (DE)
(72) Erfinder: SYFONIOS, Andreas, 97493 Bergrheinfeld (DE); STÄBLEIN, Tilman, 97072 Würzburg (DE); HÜMMER, Dirk, 97520 Hirschfeld (DE); KLÖFFEL, Peter, 97720 Nüdlingen (DE)
(74) Vertreter: Herrmann, Uwe
(86) Internationale Anmeldenummer: PCT/EP2016/001613
(87) Internationale Veröffentlichungsnummer: WO 2017/054923

(56) Entgegenhaltungen:
- EP-A1- 2 497 507
- EP-A1- 2 826 505
- EP-A2- 2 554 192
- WO-A1-2008/065470
- WO-A1-2011/112317
- WO-A1-2015/071247
- DE-C1- 4 211 455

## Beschreibung

Die vorliegende Erfindung betrifft ein Dialysegerät mit einem Dialysator, einem mit dem Dialysator und einer externen Wasserversorgung in Verbindung stehenden Wassereingangssystem zur Versorgung des Dialysators mit frischer Dialysierflüssigkeit sowie mit einer mit dem Dialysator in Verbindung stehenden Leitung für gebrauchte Dialysierflüssigkeit, wobei das Wassereingangssystem einen Behälter, im Folgenden auch als Wassereingangsbehälter bezeichnet, für Wasser, insbesondere für RO-Wasser, und einen Rezirkulationskreislauf aufweist, und wobei das Dialysegerät einen Wärmetauscher aufweist, der einerseits mit der Leitung für gebrauchte Dialysierflüssigkeit und andererseits mit dem Wassereingangssystem in Verbindung steht, so dass Wärme von der gebrauchten Dialysierflüssigkeit an das in dem Wassereingangssystem befindliche Wasser übertragen wird.

Bei aus dem Stand der Technik bekannten Dialysegeräten wird die Dialysierflüssigkeit beispielsweise dadurch hergestellt, dass frischem RO-Wasser, d.h. durch Reverse Osmose hergestelltem Wasser, ein oder mehrere Konzentrate zugegeben werden. Dieses Gemisch wird bei Bedarf dem Dialysator zugeführt.

Aus dem Stand der Technik ist beispielsweise aus der Druckschrift EP2826505A1 einen Dialyseanlage mit mehreren Dialysegeräten bekannt, welche zentral mit Wasser versorgt werden. Zudem offenbart die Druckschrift DE4211455C1 ein Verfahren zur kontinuierlichen Herstellung von Dialysierflüssigkeit.

Das zur Herstellung der Dialysierflüssigkeit verwendete RO-Wasser liegt in einem Wassereingangsbehälter vor, der bei bekannten Geräten einen Bestandteil des Rezirkulationskreislaufes darstellt.

Um die Wärme der gebrauchten Dialysierflüssigkeit nutzen zu können, ist es des Weiteren bekannt, einen Wärmetauscher einzusetzen, der auf der einen Seite von der gebrauchten Dialysierflüssigkeit durchströmt wird und auf der anderen Seite von RO-Wasser, das auf diese Weise erwärmt wird.

Da die gebrauchte Dialysierflüssigkeit durch eine Undichtigkeit in dem Wärmetauscher in das RO-Wasser und somit in das Versorgungssystem des Dialysezentrums zur zentralen Bereitstellung von RO-Wasser gelangen könnte, können Gegenmaßnahmen zur Verunreinigung des RO-Wassers zum Einsatz kommen, die eine solche Kontaminierung in jedem Falle verhindern. Eine der möglichen Gegenmaßnahmen stellt ein Sensor dar, der einen solchen Übertritt von gebrauchter Dialysierflüssigkeit erfasst.

Der Einsatz eines Systems zur Verhinderung der Kontaminierung bzw. eines Sensors und des damit verbundenen Überwachungssystems ist zwar insoweit vorteilhaft, als dass ein Leck. des Wärmetauschers zuverlässig erkannt werden kann, allerdings mit dem Nachteil eines erhöhten Kostenaufwands verbunden.

Der vorliegenden Erfindung liegt die Aufgabe zugrunde, ein Dialysegerät der eingangs genannten Art dahingehend weiterzubilden, dass auf einfache Weise sichergestellt wird, dass RO-Wasser eines zentralen Versorgungssystems nicht kontaminiert wird.

Diese Aufgabe wird durch ein Dialysegerät mit den Merkmalen des Anspruchs 1 gelöst. Danach ist vorgesehen, dass der Behälter eine Einrichtung zur physikalischen Trennung der externen Wasserversorgung und der nachgelagerten Abschnitte des Wassereingangssystems aufweist, vorzugsweise eine Freifallstrecke für eintretende Flüssigkeit von der Wasserversorgung, und dass der Wärmetauscher stromabwärts des Wassereingangsbehälters angeordnet ist. Der Begriff "stromabwärts" ist basierend auf der Strömungsrichtung des Wassers, insbesondere des RO-Wassers, bei der Herstellung der Dialysierflüssigkeit zu verstehen. Durch die physikalische Trennung des Wasserflusses innerhalb des Wassereingangsbehälters und die dem Wassereingangsbehälter und damit dieser physikalischen Barriere nachgelagerte Anordnung des Wärmetauschers kann selbst bei Auftreten einer Leckage keine Verunreinigung der externen Wasserversorgung auftreten. Somit ist die Kontaminierung der Versorgungsleitung der Klinik bzw. eines Dialysezentrums, aus der der Wassereingangsbehälter mit RO-Wasser gespeist wird, mit gebrauchter Dialysierflüssigkeit ausgeschlossen. Auch eine Kontaminierung der Versorgungsleitung, beispielsweise durch ein Desinfektionsmittel, das bei einer Reinigung des Dialysegeräts eingesetzt wird, ist somit ausgeschlossen.

In einer Ausführungsform ist kein Sensor zur Überprüfung, ob in dem Wärmetauscher ein Übertritt von gebrauchter Dialysierflüssigkeit zu der in dem Wassereingangssystem befindlichen Flüssigkeit, bei der es sich vorzugsweise um RO-Wasser handelt, erfolgt ist, vorgesehen. Auf den Sensor und die damit verbundene Auswerteeinheit kann somit verzichtet werden.

Der Begriff "Wassereingangsbehälter" umfasst jeden Behälter, der geeignet ist, das zur Herstellung der fertigen Dialysierflüssigkeit benötigte Wasser, insbesondere RO-Wasser aufzunehmen, wobei der Behälter eine Einrichtung zur physikalischen Trennung der externen Wasserversorgung und der nachgelagerten Abschnitte des Wassereingangssystems aufweist. Es kann sich dabei um einen Behälter mit festen Wandungen oder auch um einen Behälter mit flexiblen Wandungen handeln.

Der Wassereingangsbehälter stellt vorzugsweise einen Vorrat für das zur Herstellung der Dialysierflüssigkeit benötigte Wasser, wie z.B. RO-Wasser bereit. Der Wassereingangsbehälter weist vorzugsweise eine Schnittstelle auf, mittels derer RO-Wasser von der externen Wasserversorgung in den Wassereingangsbehälter eingeführt werden kann. Bei der externen Wasserversorgung kann es sich beispielsweise um ein zentrales Versorgungssystem einer Dialyseklinik handeln, in dem RO-Wasser hergestellt wird und an dem mehrere Dialysegeräte angeschlossen werden können. Bei der Schnittstelle kann es sich beispielsweise um einen Anschluss für eine Wasserzulaufleitung des Versorgungssystems handeln.

In einer Ausführungsform umfasst der Wassereingangsbehälter als physikalische Barriere eine Freifallstrecke, wobei das von der Wasserversorgung kommende Wasser unter Überwindung einer Höhendifferenz frei von einem höheren Ausgangsniveau in ein tiefer liegendes Becken fällt (z.B. fließt oder tropft), in dem das Wasser steht. Das Becken kann, beispielsweise anhand eines Siphonrohres, mit den nachgelagerten Abschnitten des Wassereingangssystems derart verbunden sein, dass das Wasser nach Überschreiten eines bestimmten Flüssigkeitslevels im Becken in die nachgelagerten Abschnitte des Wassereingangssystems fließt.

In einer bevorzugten Ausgestaltung der Erfindung weist das Wassereingangssystem einen Rezirkulationskreislauf sowie eine Leitung zwischen dem Wassereingangsbehälter und dem Rezirkulationskreislauf auf. Durch diese Leitung strömt das Wasser, insbesondere RO-Wasser aus dem Behälter in den Rezirkulationskreislauf. Der genannte Wärmetauscher befindet sich vorzugsweise in dieser Leitung und wird auf einer Seite von der gebrauchten Dialysierflüssigkeit und auf der anderen Seite von RO-Wasser oder dergleichen durchströmt.

In einer weiteren bevorzugten Ausgestaltung der Erfindung ist vorgesehen, dass das Wassereingangssystem einen Rezirkulationskreislauf aufweist und dass der Wassereingangsbehälter keinen Bestandteil des Rezirkulationskreislaufes bildet. Vorzugsweise gelangt das zur Herstellung der Dialysierflüssigkeit verwendete Wasser, vorzugsweise das RO-Wasser, somit aus dem Wassereingangsbehälter in den Wärmetauscher, in dem es erwärmt wird, und dann in den Rezirkulationskreislauf. Dadurch erhöht sich die Effektivität des Wärmetauschers.

Weiterhin kann vorgesehen sein, dass das Wassereingangssystem einen Rezirkulationskreislauf aufweist und dass das Dialysegerät ein Bilänzkammersystem aufweist, das mit dem Rezirkulationskreislauf in Verbindung steht. In diesem Fall gelangt die in dem Wassereingangssystem hergestellte Dialysierflüssigkeit von dem Rezirkulationskreislauf bzw. von den Zugabestellen für Konzentrate zur Herstellung der Dialysierflüssigkeit (unmittelbar oder mittelbar) in das Bilanzkammersystem des Dialysegerätes.

Weiterhin kann vorgesehen sein, dass das Wassereingangssystem einen Luftabscheider aufweist. Dieser hat die Aufgabe, Luft abzuscheiden, damit diese nicht in das Bilanzkammersystem sowie in den Dialysator des Dialysegerätes gelangt. Vorzugsweise ist der Luftabscheider so angeordnet, dass er aus dem RO-Wasser, das in dem Rezirkulationskreislauf zirkuliert, Luft abscheidet.

Vorzugsweise befindet sich der Luftabscheider in dem Rezirkulationskreislauf des Wassereingangssystems.

Denkbar ist es, dass eine Leitung zur Luftabscheidung zwischen dem Luftabscheider und dem Wassereingangsbehälter verläuft, wobei vorzugsweise vorgesehen ist, dass sich in der Leitung ein Ventil befindet. Mittels des Ventils kann die Leitung geöffnet und geschlossen werden, so dass eine gezielte Abfuhr von Luft aus dem Luftabscheider erfolgen kann.

Dabei ist es möglich, durch Veranlassung einer Steuer- oder Regelungseinheit dieses Ventil zyklisch zu öffnen und zu schließen.

Möglich ist es, dass die Steuer- oder Regelungseinheit derart ausgebildet ist, dass das Ventil zu einem bestimmten Zeitpunkt und/oder für eine bestimmte Zeitspanne öffnet und/oder schließt.

In einer weiteren Ausgestaltung der Erfindung ist vorgesehen, dass das Wassereingangssystem ein Zudosierungssystem aufweist, mittels dessen ein oder mehrere Konzentrate zugebbar sind. So können dem vorgelegten Wasser, insbesondere RO-Wasser ein oder mehrere Konzentrate zugegeben werden, die zur Herstellung der fertigen Dialysierflüssigkeit benötigt werden. Dabei besteht eine bevorzugte Ausgestaltung der Erfindung darin, dass das Wassereingangssystem einen Rezirkulationskreislauf aufweist und das oder die Konzentrate aus dem oder den Konzentratbehältern durch eine oder mehrere Leitungen dem aus dem Rezirkulationskreislauf ausströmenden RO-Wasser zuführbar sind.

Denkbar ist es, dass das Zudosierungssystem mit dem Luftabscheider in Verbindung steht, so dass das oder die Konzentrate in den Luftabscheider eingeführt werden. Möglich ist es, dass das Zudosierungssystem eine oder mehrere Leitungen umfasst, von denen wenigstens eine in einen unteren Bereich des Luftabscheiders mündet.

Der Luftabscheider kann einen ersten Abschnitt und einen zweiten Abschnitt aufweisen, wobei der erste Abschnitt unterhalb des zweiten Abschnitts angeordnet ist und wobei das Zudosierungssystem mit dem unteren Abschnitt und die genannte Leitung mit dem oberen Abschnitt des Luftabscheiders in Verbindung steht.

Bevorzugt ist es, wenn der untere Abschnitt des Luftabscheiders mit dem Bilanzkammersystem des Gerätes in Verbindung steht. Von dort aus gelangt die fertige Dialysierflüssigkeit in das Bilanzkammersystem des Gerätes.

Der Luftabscheider kann eine Trennplatte mit einer oder mehreren Öffnungen aufweisen, die den ersten von dem zweiten Abschnitt des Luftabscheiders trennt. Die Trennplatte verhindert, dass das oder die zugegebenen Konzentrate in den oberen Teil des Luftabscheiders gelangen. Der obere Teil des Luftabscheiders ist Teil des Rezirkulationskreislaufes. Der untere Teil des Luftabscheiders ist nicht Teil des Rezirkulationskreislaufes und dient als Zugabestelle für das wenigstens eine Konzentrat und zur Mischung des Konzentrats mit dem RO-Wasser. Diese Mischung gelangt dann zum Dialysator bzw. zu dem diesem vorgelagerten Bilanziersystem. Daher bleibt das in dem Rezirkulationskreislauf befindliche Fluid, insbesondere RO-Wasser konzentratfrei.

Eine weitere Funktion der Trennplatte besteht darin, dass Luft, die von den Konzentratpumpen herrühren kann und in dem oder den Konzentraten enthalten ist (z.B. aufgrund eines leeren Konzentratkanisters) durch die Bohrungen der Trennplatte nach oben steigt und so in den Rezirkulationskreislauf gelangt, in dem sie abgeschieden wird.

Das Wassereingangssystem kann einen Rezirkulationskreislauf aufweisen, wobei Mittel vorgesehen sind, die derart ausgebildet sind, dass der Rezirkulationskreislauf aus dem Wassereingangsbehälter nachgefüllt wird, wenn eine Entnahme von Wasser aus dem Rezirkulationskreislauf zu dem Dialysator bzw. zu dem Bilanzkammersystem erfolgt ist. Diese Mittel können derart ausgebildet sein, dass das Volumen ermittelt wird, das dem Rezirkulationskreislauf entnommen wird und ein entsprechendes Volumen aus dem Wassereingangsbehälter nachgefüllt wird. Auch ist es möglich, dass die Mittel derart ausgebildet sind, dass der Druck in dem Rezirkulationskreislauf, der nach der Entnahme von RO-Wasser zur Herstellung der Dialysierflüssigkeit gesunken ist, durch Nachfüllen des Rezirkulationskreislaufes wieder aufgebaut, d.h. erhöht wird. Ein Druckabfall im Rezirkulationskreislauf aufgrund einer Entnahme von fertiger Dialysierflüssigkeit wird somit dadurch kompensiert, dass Fluid in einer Menge aus dem Wassereingangsbehälter in den Rezirkulationskreislauf nachgefüllt wird, so dass der Druck wieder aufgebaut wird.

Die vorliegenden Erfindung betrifft des Weiteren ein Dialysegerät mit einem Dialysator sowie mit einem mit dem Dialysator und einer externen Wasserversorgung in Verbindung stehenden Wassereingangssystem zur Versorgung des Dialysators mit frischer Dialysierflüssigkeit, wobei das Wassereingangssystem einen Rezirkulationskreislauf zur Rezirkulation des zur Herstellung der Dialysierflüssigkeit benötigen Wassers, insbesondere von RO-Wasser aufweist, wobei in dem Rezirkulationskreislauf ein Luftabscheider angeordnet ist, von dem eine Leitung zur Luftabscheidung abgeht und zu dem eine Leitung zur Zufuhr eines Konzentrats verläuft, die sich zwischen einem Zudosierungssystem und dem Luftabscheider erstreckt.

Der Luftabscheider hat somit nicht nur die Funktion der Luftabscheidung, sondern dient ferner zur Aufnahme des oder der Konzentrate bzw. zur Durchmischung des oder der Konzentrate mit dem in dem Rezirkulationskreislauf strömenden Wasser, insbesondere mit RO-Wasser.

Das Dialysegerät gemäß diesem Aspekt der Erfindung kann gemäß einem oder mehreren der Ansprüche 1 bis 15 ausbildet sein. So ist es beispielsweise denkbar, dass der Luftabscheider eine Trennplatte aufweist, die eine oder mehrere Bohrungen aufweist, wobei sich die Leitung zur Zufuhr von Konzentrat unterhalb der Trennplatte und die Leitung zur Abfuhr von Luft oberhalb der Trennplatte befindet. Vorzugsweise ist nur der Bereich oberhalb der Trennplatte Bestandteil des Rezirkulationskreislaufes.

An dieser Stelle wird darauf hingewiesen, dass die Verwendung der Begriffe "ein/eine" nicht dahingehend einschränkend auszulegen ist, dass genau eines der betreffenden Elemente vorhanden ist. Vielmehr umfassen die Begriffe auch das Vorhandensein von zwei oder mehr als zwei der fraglichen Elemente.

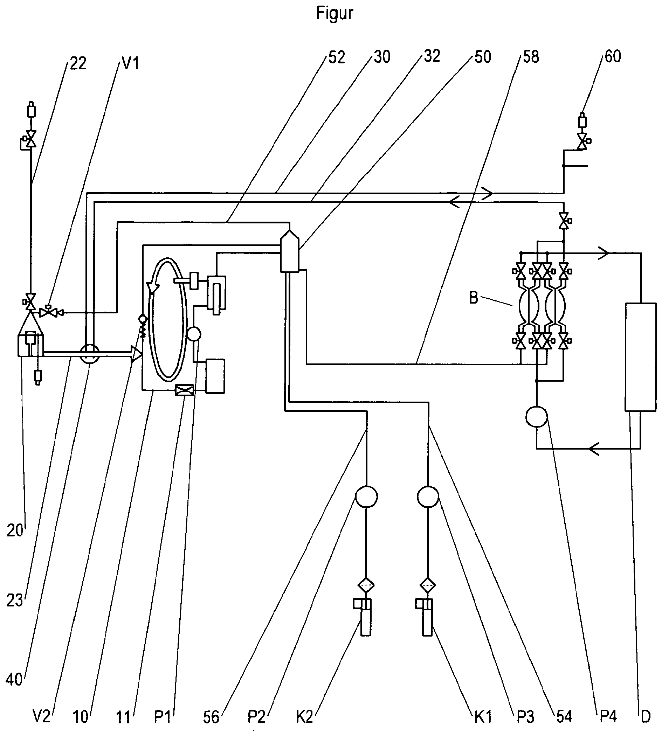

Weitere Einzelheiten und Vorteile der Erfindung werden anhand eines in der Zeichnung dargestellten Ausführungsbeispiels näher erläutert.

Die einzige Figur zeigt eine schematische Ansicht eines hydraulischen Systems eines Dialysegerätes gemäß der Erfindung.

Die Figur zeigt ein hydraulisches System eines Dialysegerätes gemäß der Erfindung. Das hydraulische System umfasst den Wassereingangsbehälter bzw. Behälter 20, in dem sich RO-Wasser befindet.

Der Behälter 20 wird über die Wasserlaufstrecke 22, welche beispielsweise einem zentralen Versorgungssystem für RO-Wasser eines Dialysezentrums zuzuordnen ist, mit RO-Wasser befüllt.

Das Bezugszeichen 10 kennzeichnet einen Rezirkulations- und Entgasungskreislauf, in dem eine Lösung bzw. eine Flüssigkeit, insbesondere RO-Wasser, die zur Herstellung der fertigen Dialysierflüssigkeit dient, mittels der Pumpe P1 zirkuliert. Neben weiteren Komponenten sind ein Druckbegrenzungsventil V2 sowie der Primärluftabscheider 50 in dem Rezirkulationskreislauf 10 angeordnet. In dem Rezirkulationskreislauf befindet sich kein Konzentrat. Dieses wird erst stromabwärts des Rezirkulationskreislaufes zugegeben.

In dem Rezirkulationskreislauf wird das RO-Wasser entgast und beheizt.

Wie dies aus der Figur weiter hervorgeht, verläuft eine Leitung 23 zwischen dem Behälter 20 und dem Rezirkulationskreislauf 10. Über diese Leitung 23 wird das in dem Behälter 10 befindliche Fluid, insbesondere RO-Wasser, dem Rezirkulationskreislauf 10 zugeleitet.

Wie dies weiter aus der Figur hervorgeht, verläuft von dem Primärluftabscheider 50 zu dem Wassereingangsbehälter 20 eine Leitung 52, die durch ein Ventil V1 verschließbar ist und bei Bedarf geöffnet wird. Diese Leitung 52 dient zur Abfuhr von Luft aus dem Primärluftabscheider über das Ventil V1 in den Behälter 20. Durch die Abscheidung von Luft soll sichergestellt werden, dass das in dem Rezirkulationskreislauf befindliche Fluid bzw. RO-Wasser luftfrei ist.

Die Luft wird freigesetzt durch die Entgasung des RO-Wassers, indem beispielsweise durch die Entgasungspumpe P1 in Verbindung mit der Entgasungsdrossel 11 ein Unterdruck erzeugt wird, der zum Entgasen des RO-Wassers führt. Eine weitere Quelle für Luft besteht darin, dass einer der Konzentratbehälter K1, K2 leer ist. Alternativ oder zusätzlich kann beispielsweise vorgesehen sein, das Wasser durch Erhitzen zu entgasen.

Die Leitungen 30 und 32 kennzeichnen Leitungen für gebrauchte Dialysierflüssigkeit, d.h. Leitungen, die mit dem Dialysator in Verbindung stehen und durch die gebrauchte Dialysierflüssigkeit vom Dialysator strömt. Über die Leitung 32 gelangt die gebrauchte Dialysierflüssigkeit zu dem Wärmetauscher 40 und von diesem über die Leitung 30 zu einem Abfluss 60.

Das Bezugszeichen B kennzeichnet das Bilanziersystem, mittels dessen sichergestellt wird, dass die zu dem Dialysator geförderte Dialysierflüssigkeit in demselben Volumen zugeführt wird, wie von dem Dialysator gelangende gebrauchte Dialysierflüssigkeit abgeführt wird. Das Bezugszeichen D kennzeichnet den Dialysator, der eine Mehrzahl von Hohlfasermembranen aufweist, die auf der einen Seite von Dialysierflüssigkeit und auf der anderen Seite von Blut umströmt bzw. durchströmt werden. Wie dies aus der Figur hervorgeht, steht der Dialysator D eingangsseitig und auslassseitig mit dem Bilanziersystem B in Verbindung. Das Bezugszeichen P4 kennzeichnet die Dialysierflüssigkeitspumpe, die die Dialysierflüssigkeit fördert. Diese ist dem hier gezeigten Ausführungsbeispiel stromabwärts des Dialysators D angeordnet.

Die Bezugszeichen K1 und K2 kennzeichnen vereinfacht ausgedrückt Konzentratbehälter, bspw. für ein basisches und ein saures Konzentrat. Diese Behälter stehen über Leitungen 54, 56 mit dem unteren Abschnitt bzw. mit dem Boden des Primärluftabscheiders 50 in Verbindung. Die Förderung der Konzentrate aus den Konzentratbehältern K1 und K2 erfolgt mittels der Pumpen P2 und P3 durch die Leitungen 54 und 56. Vorzugsweise handelt es sich bei den Bezugszeichen K1 und K2 um Spülkammern für Konzentratansaugstäbe.

Die Zirkulation im Rezirkulationskreislauf 10 ist durch den geschlossenen Pfeil in der Mitte des Rezirkulationskreislaufes dargestellt.

Wie dies aus der Figur hervorgeht, befindet sich der Wärmetauscher 40 stromabwärts des Wassereingangsbehälters 20 und zwar zwischen dem Wassereingangsbehälter 20 und dem Rezirkulationskreislauf 10.

Der Wassereingangsbehälter 20 weist eine Freifallstrecke auf, in der von der zentralen Wasserversorgung über die Wasserzulaufstrecke 22 in den Behälter 20 laufendes RO-Wasser von einem höheren Ausgangsniveau frei durch die Luft in ein tiefer liegendes Becken fällt. Das Becken ist anhand eines Siphonrohres mit der Leitung 23 verbunden. Durch diese Ausbildung fließt die Flüssigkeit nach Überschreiten eines bestimmten Flüssigkeitslevels im Becken in die Leitung 23. Selbstverständlich sind auch andere Einrichtungen zur Einstellung des Flüssigkeitsniveaus im Becken bzw. zur Flussbegrenzung in die Leitung 23 denkbar.

Abweichend von aus dem Stand der Technik bekannten Anordnungen, bei denen der Wärmetauscher vor, d.h. stromaufwärts, des Wassereingangsbehälters platziert ist, besteht erfindungsgemäß keine Notwendigkeit, den Wärmetauscher 40 dahingehend zu überprüfen, ob eine Leckage vorliegt, wegen der aus der Leitung 32 gebrauchte Dialysierflüssigkeit in die Wasserzulaufstrecke 22 und damit in die zentrale Versorgungseinrichtung des Dialysezentrums gelangt.

Im Betrieb der dargestellten Anordnung wird dem Rezirkulationskreislauf über die Leitung 23 RO-Wasser und dem unteren Teil des Primärluftabscheiders 50 über die Leitungen 54 und 56 Konzentrate zugeführt. Mittels Förderung durch die Pumpe P1 werden diese Komponenten zu einer fertigen Dialysierflüssigkeit gemischt.

Wird die fertige Dialysierflüssigkeit für die Behandlung benötigt, strömt sie aus dem unteren Bereich des Primärluftabscheiders 50 über die Leitung 58 in das Bilanzkammersystem B. Über die Leitung 23 strömt ein entsprechend großes Volumen RO-Wassers in den Rezirkulationskreislauf 10 nach.

Wie dies aus der Figur hervorgeht, erfolgt die Rezirkulation der Flüssigkeit in dem Rezirkulationskreislauf 10 nicht über die Wassereingangskammer 20, da diese dem Rezirkulationskreislauf 10 vorgeschaltet ist und keinen Bestandteil von diesem bildet. Stattdessen erfolgt die Rezirkulation nach, d.h. stromabwärts des Wärmetauschers 40 über ein Druckbegrenzungsventil V2 in einem separatem Wassereingangskreis 10.

Wie dies oben ausgeführt wurde, erfolgt die Verbindung zwischen dem Primärluftabscheider 50 in dem separatem Wassereingangskreis 10 und der Wassereingangskammer 20 über ein Ventil V1. Die dadurch erfolgte Luftabscheidung kann zyklisch (zu einem bestimmten Zeitpunkt) über das Ventil, ggf. mit bestimmter Öffnungszeit, in die Wassereingangskammer 20 erfolgen.

Der separate Wassereingangskreis, der oben auch als Rezirkulationskreislauf 10 bezeichnet wird, versorgt die Bilanzkammer B des Gerätes nach Zugabe des oder der Konzentrate mit temperierter, gemischter Dialysierflüssigkeit.

Wird - wie ausgeführt - Lösung aus dem Rezirkulationskreislauf 10 entnommen, d.h. zum Bilanzkammersystem B geführt, wird die entnommene Menge aus der Wassereingangskammer 20 nachgefüllt und über den Wärmetauscher 40 mittels der gebrauchten Dialysierflüssigkeit vorgewärmt.

Wie dies aus der Figur weiter hervorgeht, erfolgt die Luftabscheidung aus dem Primärluftabscheider 50 in dessen oberen Bereich, der abweichend von dem unteren Bereich mit dem Rezirkulationskreislauf verbunden ist bzw. dessen Bestandteil bildet. Die Zufuhr von Konzentraten sowie die Abfuhr der fertigen Dialysierflüssigkeit mittels der Leitungen 54, 56 und 58 erfolgt aus einem dem gegenüber unteren Abschnitt des Primärluftabscheiders.

Diese beiden Abschnitte des Primärluftabscheiders 50 sind mittels einer Trennplatte mit Öffnungen für die Luftabscheidung (im Fehlerfall bei leerem Kanister) miteinander verbunden.

Durch die Anordnung des Wärmetauschers 40 nach dem Wassereingangs-behälter 20 mit einer Freifallstrecke oder einer anderen Einrichtung zur physikalischen Trennung des nachgelagerten hydraulischen Systems des Dialysegerätes von der Versorgungsleitung 22 kann auf eine vergleichsweise teure Überwachung einer Leckage des Wärmetauschers 40 verzichtet werden, wobei gleichzeitig die Effektivität des Wärmetauschers garantiert wird. In der hier dargestellten Anordnung dient der Wärmetauscher dazu, das RO-Wasser, das von der Wassereingangskammer 20 zu dem Kreislauf 10 strömt, zu erwärmen. Dazu kann der Wärmetauscher 40 eine Primärseite und eine Sekundärseite aufweisen, wobei die Primärseite durch die gebrauchte Dialysierflüssigkeit und die Sekundärseite durch das RO-Wasser durchströmt wird.

## Patentansprüche

1. Dialysegerät mit einem Dialysator (D), einem mit dem Dialysator und einer externen Wasserversorgung in Verbindung stehenden Wassereingangssystem zur Versorgung des Dialysators (D) mit frischer Dialysierflüssigkeit sowie mit einer mit dem Dialysator (D) in Verbindung stehenden Leitung (32) für gebrauchte Dialysierflüssigkeit, wobei das Wassereingangssystem einen Behälter (20) für Wasser, insbesondere für RO-Wasser aufweist, und wobei das Dialysegerät des Weiteren einen Wärmetauscher (40) aufweist, der einerseits mit der Leitung (32) für gebrauchte Dialysierflüssigkeit und andererseits mit dem Wassereingangssystem in Verbindung steht, so dass Wärme von der gebrauchten Dialysierflüssigkeit an das in dem Wassereingangssystem befindliche Wasser übertragen wird,
**dadurch gekennzeichnet,**
**dass** der Behälter (20) eine Einrichtung zur physikalischen Trennung der externen Wasserversorgung und der nachgelagerten Abschnitte des Wassereingangssystems aufweist, vorzugsweise eine Freifallstrecke für eintretende Flüssigkeit von der Wasserversorgung, und dass der Wärmetauscher (40) stromabwärts des Behälters (20) angeordnet ist.

2. Dialysegerät nach Anspruch 1, **dadurch gekennzeichnet, dass** kein Sensor vorgesehen ist, mittels dessen überprüfbar ist, ob in dem Wärmetauscher (40) ein Übertritt von gebrauchter Dialysierflüssigkeit zu dem in dem Wassereingangssystem befindlichen Wasser erfolgt.

3. Dialysegerät nach Anspruch 1 oder 2, **dadurch gekennzeichnet, dass** das Wassereingangssystem einen Rezirkulationskreislauf (10) sowie eine Leitung (23) zwischen dem Behälter (20) und dem Rezirkulationskreislauf (10) aufweist und dass sich der Wärmetauscher (40) in dieser Leitung (23) selbst befindet.

4. Dialysegerät nach einem der vorhergehenden Ansprüche, **dadurch gekennzeichnet, dass** das Wassereingangssystem einen Rezirkulationskreislauf (10) aufweist und dass der Behälter (20) keinen Bestandteil des Rezirkulationskreislaufes (10) bildet.

5. Dialysegerät nach einem der vorhergehenden Ansprüche, **dadurch gekennzeichnet, dass** das Wassereingangssystem einen Rezirkulationskreislauf (10) aufweist und dass das Dialysegerät ein Bilanzkammersystem (B) aufweist, das mit dem Rezirkulationskreislauf (10) in Verbindung steht, so dass die Dialysierflüssigkeit von dem Rezirkulationskreislauf (10) zu dem Bilanzkammersystem (B) strömt.

6. Dialysegerät nach einem der vorhergehenden Ansprüche, **dadurch gekennzeichnet, dass** das Wassereingangssystem einen Luftabscheider (50) aufweist, der sich vorzugsweise in einem Rezirkulationskreislauf (10) des Wassereingangssystems befindet.

7. Dialysegerät nach Anspruch 6, **dadurch gekennzeichnet, dass** eine Leitung (52) zur Luftabscheidung zwischen dem Luftabscheider (50) und dem Behälter (20) verläuft, wobei vorzugsweise vorgesehen ist, dass sich in der Leitung (52) ein Ventil (V1) befindet.

8. Dialysegerät nach Anspruch 7, **dadurch gekennzeichnet, dass** das Dialysegerät eine Steuer- oder Regelungseinheit aufweist, die derart ausgebildet ist, dass diese das Ventil (V1) zyklisch öffnet und schließt.

9. Dialysegerät nach Anspruch 8, **dadurch gekennzeichnet, dass** die Steuer- oder Regelungseinheit derart ausgebildet ist, dass diese das Ventil (V1) zu einem bestimmten Zeitpunkt und/oder für eine bestimmte Zeitspanne öffnet und/oder schließt.

10. Dialysegerät nach einem der vorhergehenden Ansprüche, **dadurch gekennzeichnet, dass** das Wassereingangssystem ein Zudosierungssystem aufweist, mittels dessen ein oder mehrere Konzentrate zu dem Wasser zur Herstellung der Dialysierflüssigkeit zugebbar sind, wobei vorzugsweise vorgesehen ist, dass das Wassereingangssystem einen Rezirkulationskreislauf (10) aufweist und das Zudosierungssystem derart ausgebildet ist, dass das oder die Konzentrate stromabwärts des Rezirkulationskreislaufes (10) zugebbar sind.

11. Dialysegerät nach einem der Ansprüche 6 bis 10, **dadurch gekennzeichnet, dass** das Zudosierungssystem mit dem Luftabscheider (50) in Verbindung steht, so dass das oder die Konzentrate in den Luftabscheider (50) eingeführt werden können.

12. Dialysegerät nach Anspruch 6 und 11, **dadurch gekennzeichnet, dass** der Luftabscheider (50) einen ersten Abschnitt und einen zweiten Abschnitt aufweist, wobei der erste Abschnitt unterhalb des zweiten Abschnittes angeordnet ist und wobei das Zudosierungssystem mit dem unteren Abschnitt und die genannte Leitung (52) zur Luftabscheidung mit dem oberen Abschnitt des Luftabscheiders (50) in Verbindung steht.

13. Dialysegerät nach Anspruch 12, **dadurch gekennzeichnet, dass** der untere Abschnitt des Luftabscheiders (50) mit dem Bilanzkammersystem (B) des Dialysegerätes in Verbindung steht und/oder dass der obere Abschnitt des Luftabscheiders (50) einen Bestandteil des Rezirkulationskreislaufes (10) bildet.

14. Dialysegerät nach Anspruch 12 oder 13, **dadurch gekennzeichnet, dass** der Luftabscheider (50) eine Trennplatte mit einer oder mehreren Öffnungen aufweist, die den ersten von dem zweiten Abschnitt des Luftabscheiders (50) trennt.

15. Dialysegerät nach einem der vorhergehenden Ansprüche, **dadurch gekennzeichnet, dass** das Wassereingangssystem einen Rezirkulationskreislauf (10) aufweist und dass Mittel vorgesehen sind, die derart ausgebildet sind, dass der Rezirkulationskreislauf (10) aus dem Behälter (20) nachgefüllt wird, wenn eine Entnahme von Wasser aus dem Rezirkulationskreislauf (10) erfolgt ist.

## Claims

1. Dialysis machine with a dialyzer (D), a water inlet system for supplying the dialyzer (D) with fresh dialysis fluid which is connected to the dialyzer and an external water supply, and with a line (32) in communication with the dialyzer (D) for used dialysis fluid, wherein the water inlet system comprises a container (20) for water, in particular for RO water, and wherein the dialysis machine further comprises a heat exchanger (40), being in communication on the one hand with the line (32) for used dialysis fluid and on the other hand with the water inlet system so that heat is transferred from the used dialysis fluid to the fluid in the water inlet system,
**characterized in**
**that** the container (20) comprises means for physical separation of the external water supply and the downstream sections of the water inlet system, preferably a free-fall path for incoming liquid from the water supply, and that the heat exchanger (40) is arranged downstream of the container (20).

2. Dialysis machine according to claim 1, **characterized in that** no sensor is provided by means of which it is possible to check whether a transfer of used dialysis fluid to the water present in the water inlet system takes place in the heat exchanger (40).

3. Dialysis machine according to claim 1 or 2, **characterized in that** the water inlet system comprises a recirculation circuit (10) and a line (23) between the container (20) and the recirculation circuit (10) and that the heat exchanger (40) itself is located in this line (23).

4. Dialysis machine according to one of the preceding claims, **characterized in that** the water inlet system comprises a recirculation circuit (10) and that the container (20) forms no part of the recirculation circuit (10).

5. Dialysis machine according to one of the preceding claims, **characterized in that** the water inlet system comprises a recirculation circuit (10) and that the dialysis machine comprises a balancing chamber system (B) which communicates with the recirculation circuit (10), so that the dialysis fluid flows from the recirculation circuit (10) to the balancing chamber system (B).

6. Dialysis machine according to one of the preceding claims, **characterized in that** the water inlet system comprises an air separator (50), which is preferably located in a recirculation circuit (10) of the water inlet system.

7. Dialysis machine according to claim 6, **characterized in that** a line (52) for air separation extends between the air separator (50) and the container (20), wherein it is preferably provided that in the conduit (52) a valve (V1) is located.

8. Dialysis machine according to claim 7, **characterized in that** the dialysis machine comprises a control or regulating unit which is designed such that it cyclically opens and closes the valve (V1).

9. Dialysis machine according to claim 8, **characterized in that** the control or regulating unit is designed such that it opens and / or closes the valve (V1) at a certain time and / or for a certain period of time.

10. Dialysis machine according to one of the preceding claims, **characterized in that** the water inlet system comprises a dosage system, by means of which one or more concentrates may be added to the water for producing the dialysis fluid, wherein it is preferably provided that the water inlet system comprises a recirculation circuit (10) and the dosage system is designed such that the concentrate or concentrates may be added downstream of the recirculation circuit (10).

11. Dialysis machine according to one of claims 6 to 10, **characterized in that** the dosage system is in communication with the air separator (50) so that the concentrate or concentrates can be introduced into the air separator (50).

12. Dialysis machine according to claim 6 and 11, **characterized in that** the air separator (50) comprises a first portion and a second portion, wherein the first portion is arranged below the second portion and wherein the dosage system is in communication with the lower portion and said line (52) for air separation is in communication with the upper portion of the air separator (50).

13. Dialysis machine according to claim 12, **characterized in that** the lower portion of the air separator (50) is in communication with the balancing chamber system (B) of the dialysis machine and / or that the upper portion of the air separator (50) forms a part of the recirculation circuit (10).

14. Dialysis machine according to claim 12 or 13, **characterized in that** the air separator (50) comprises a partition plate with one or more openings, which separates the first from the second section of the air separator (50).

15. Dialysis machine according to one of the preceding claims, **characterized in that** the water inlet system comprises a recirculation circuit (10) and that means are provided which are designed such that the recirculation circuit (10) is refilled from the container (20) when a removal of water from the recirculation circuit (10) has been carried out.

## Revendications

1. Appareil de dialyse comprenant un dialyseur (D), un système d'admission d'eau raccordé au dialyseur et une alimentation externe en eau pour alimenter le dialyseur (D) en fluide de dialyse frais, ainsi qu'une tubulure (32), raccordée au dialyseur (D), pour le fluide de dialyse usagé, le système d'admission d'eau comportant un récipient (20) pour de l'eau, en particulier pour de l'eau osmosée, et l'appareil de dialyse comportant en outre un échangeur de chaleur (40), qui est raccordé d'une part à la tubulure (32) pour le fluide de dialyse usagé et d'autre part au système d'admission d'eau, de telle sorte que de la chaleur est transférée du fluide de dialyse usagé à l'eau se trouvant dans le système d'admission d'eau,
**caractérisé en ce que**
le récipient (20) comporte un système destiné à la séparation physique entre l'alimentation externe en eau et les parties en aval du système d'admission d'eau, de préférence une section de chute libre pour du liquide entrant depuis l'alimentation en eau, et **en ce que** l'échangeur de chaleur (40) est disposé en aval du récipient (20).

2. Appareil de dialyse selon la revendication 1, **caractérisé en ce qu'**aucun capteur n'est prévu, au moyen duquel il est possible de vérifier si, dans l'échangeur de chaleur (40), un passage de fluide de dialyse usagé se fait vers l'eau se trouvant dans le système d'admission d'eau.

3. Appareil de dialyse selon la revendication 1 ou 2, **caractérisé en ce que** le système d'admission d'eau comporte un circuit de recirculation (10) ainsi qu'une tubulure (23) entre le récipient (20) et le circuit de recirculation (10) et **en ce que** l'échangeur de chaleur (40) se trouve dans cette même tubulure (23).

4. Appareil de dialyse selon l'une des revendications précédentes, **caractérisé en ce que** le système d'admission d'eau comporte un circuit de recirculation (10) et **en ce que** le récipient (20) ne fait pas partie du circuit de recirculation (10).

5. Appareil de dialyse selon l'une des revendications précédentes, **caractérisé en ce que** le système d'admission d'eau comporte un circuit de recirculation (10) et **en ce que** l'appareil de dialyse comporte un système de chambre d'équilibrage (B), qui est raccordé au circuit de recirculation (10), de telle sorte que le fluide de dialyse s'écoule du circuit de recirculation (10) au système de chambre d'équilibrage (B).

6. Appareil de dialyse selon l'une des revendications précédentes, **caractérisé en ce que** le système d'admission d'eau comporte un séparateur d'air (50), qui se trouve de préférence dans un circuit de recirculation (10) du système d'admission d'eau.

7. Appareil de dialyse selon la revendication 6, **caractérisé en ce qu'**une tubulure (52) pour la séparation d'air s'étend entre le séparateur d'air (50) et le récipient (20), une soupape (V1) se trouvant de préférence dans la tubulure (52).

8. Appareil de dialyse selon la revendication 7, **caractérisé en ce que** l'appareil de dialyse comporte une unité de commande ou de régulation, qui est conçue de manière à ouvrir et fermer la soupape (V1) de manière cyclique.

9. Appareil de dialyse selon la revendication 8, **caractérisé en ce que** l'unité de commande ou de régulation est conçue de manière à ouvrir et/ou fermer la soupape (V1) à un moment précis et/ou pour une durée précise.

10. Appareil de dialyse selon l'une des revendications précédentes, **caractérisé en ce que** le système d'admission d'eau comporte un système d'addition dosée, au moyen duquel un ou plusieurs concentrés peuvent être ajoutés à l'eau pour fabriquer le fluide de dialyse, le système d'admission d'eau comportant de préférence un circuit de recirculation (10) et le système d'addition dosée étant conçu de telle manière que le ou les concentrés peuvent être ajoutés en aval du circuit de recirculation (10).

11. Appareil de dialyse selon l'une des revendications 6 à 10, **caractérisé en ce que** le système d'addition dosée est raccordé au séparateur d'air (50), de telle sorte que le ou les concentrés peuvent être introduits dans le séparateur d'air (50).

12. Appareil de dialyse selon les revendications 6 et 11, **caractérisé en ce que** le séparateur d'air (50) comporte une première partie et une seconde partie, la première partie étant disposée en dessous de la seconde partie et le système d'addition dosée étant raccordé à la partie inférieure et ladite tubulure (52) pour la séparation d'air à la partie supérieure du séparateur d'air (50).

13. Appareil de dialyse selon la revendication 12, **caractérisé en ce que** la partie inférieure du séparateur d'air (50) est raccordée au système de chambre d'équilibrage (B) de l'appareil de dialyse et/ou **en ce que** la partie supérieure du séparateur d'air (50) fait partie du circuit de recirculation (10).

14. Appareil de dialyse selon la revendication 12 ou 13, **caractérisé en ce que** le séparateur d'air (50) comporte une plaque séparatrice dotée d'une ou plusieurs ouvertures, qui sépare la première et la seconde partie du séparateur d'air (50).

15. Appareil de dialyse selon l'une des revendications précédentes, **caractérisé en ce que** le système d'admission d'eau comporte un circuit de recirculation (10) et **en ce que** des moyens sont prévus, qui sont conçus de telle manière que le circuit de recirculation (10) est rempli à partir du récipient (20) quand un prélèvement d'eau a eu lieu à partir du circuit de recirculation (10).
